# EUROPEAN PATENT APPLICATION

(11) **EP 3 671 760 A1**
(43) Date of publication of application: **24.06.2020**
(21) Application number: 18214208.3
(22) Date of filing: 19.12.2018
(51) Int. Cl.: G16H 50/30

(54) **SYSTEM AND METHOD FOR PREDICTING AN EXACERBATION**

(71) Applicant: Linde GmbH, 82049 Pullach (DE)
(72) Inventor: REID, Dennis, West Sussex, RH20 1AS (GB); JACOBSON, Brian, London, SW12 9RF (GB); THIND, Mandip, London, UB2 5RS (GB)

(57) **Abstract**

The present invention pertains to a system and a corresponding method for predicting an exacerbation of a health condition of a patient. Accordingly, a system (1) is suggested comprising an activity sensor unit (2) for measuring activity levels (20) of a patient (100), a sensor (3) arranged to be in fluid communication with a medical gas flow between a medical gas source (10) and the patient (100) and configured to measure a respiratory parameter (30) of the patient (100), and an evaluation unit (4) in communication with the activity sensor (2) and the sensor (3), wherein the evaluation unit (4) is configured to indicate a probability of an exacerbation based on the measured activity level (20) and the measured respiratory parameter (30).

## Description

### Technical Field

The invention relates to a device system for predicting an exacerbation of a health condition of a patient, for example a patient having a respiratory disorder, such as COPD. Furthermore, the invention relates to a corresponding method for predicting an exacerbation of a health condition of a patient.

### Technological Background

Patients with an impaired respiratory function are often prescribed with a medical gas therapy. One of the most common respiratory diseases worldwide is chronic obstructive pulmonary disease (COPD) which is rising in incidence and prevalence and it is a major cause of mortality. As a therapeutic measure, patients from such respiratory diseases are generally prescribed long term oxygen therapy (LTOT), which is preferably provided at a location remote from a medical facility, preferably at the patient's home. Such therapy can be provided from a number of medical gas sources including gaseous oxygen (GOX), liquid oxygen (LOX), or is provided by e.g. oxygen concentrators.

Generally, patients receiving long term oxygen therapy can experience a deterioration in symptoms which leads to an exacerbation or worsening of the disease status and often results in a hospital admission. Once a patient has experienced an exacerbation, even when they may eventually recover, the patient's health is generally reduced or in a poorer condition, thereby potentially further aggravating the patient's condition, leading to a downward spiral in patient health.

In addition to this significant morbidity, COPD constitutes a major health economic burden for both developed and developing countries since hospital admissions are extremely costly for the care provider- Accordingly, it would be advantageous if deteriorations in patient health can be detected at an early stage so as to avoid exacerbations and hospital admissions while furthermore providing an improved patient outcome.

In order to be able to predict patient exacerbations methods and systems are known, which monitor trends in the respiratory rate over time. When the respiratory rate exceeds a predefined threshold, an alert may be triggered either locally or remotely, indicating that the patient may require an intervention by a medical specialist. However, the exacerbation prediction can be difficult to predict as the patient's health is generally defined by more than one parameter and is hence influenced by several external factors. Accordingly, when a prediction is based on a measurement of a respiratory rate alone, the validity or correctness of the indication may be subject to error and the tolerance level may be accordingly adjusted. For example, it may comprise a rather narrow range to avoid a potential alert in the absence of an exacerbation, thereby potentially missing a detection of an actual exacerbation, or may comprise a large range to include every potential exacerbation while accordingly increasing the number of - potentially unnecessary - interventions.

Furthermore, such measurements are normally performed either at a routine visit of a patient to his general practitioner or in a hospital, wherein the patient is at rest and hence only provides a "baseline" measurement. Further, it is generally considered impractical to conduct such measurements in the patient's home while administering the test in hospitals or clinics is expensive, inconvenient and requires considerable effort, increasing the burden on the patient.

### Summary of the invention

Starting from the known prior art, it is an object of the present invention to provide a system and a method for predicting an exacerbation of a health condition of a patient, which provide an improved accuracy of the exacerbation prediction.

Said object is achieved by a system for predicting an exacerbation of a health condition of a patient, for example a patient having a respiratory disorder, such as COPD, according to the features of claim 1. Preferred embodiments are defined in the dependent claims, the Figures, as well as the present description.

Accordingly, a system for predicting an exacerbation of a health condition of a patient is suggested, comprising an activity sensor unit for measuring activity levels of a patient, a sensor arranged to be in fluid communication with a medical gas flow between a medical gas source and the patient and configured to measure a respiratory parameter of the patient, and an evaluation unit in communication with the activity sensor and the sensor. The evaluation unit is furthermore configured to indicate a probability of an exacerbation based on the measured activity level and the measured respiratory parameter.

Although the system is of particular relevance for patients having respiratory disorders, in particular COPD patients, the system may also be used for medical gas applications for patients suffering from other diseases and which are at risk of developing an exacerbation. For example, patients suffering from cardiovascular disorders or heart diseases may require an increased oxygen respiration and intake while in general patients may be provided with gaseous analgesic agents for a variety of diseases, dysfunctions, disorders, and/or treatments.

A worsening of the patient's health status or condition generally results in a change of physiological parameters and a corresponding change in a breathing pattern. Accordingly, by measuring the respiratory parameter, the system may detect a change in the patient's breathing pattern and associate such a change with an indication of a changed health status. This is particularly the case for patients suffering from respiratory diseases, such as COPD, cystic fibrosis, or pulmonary infections.

By the same token, a change in activity of the patient may alter the cardiovascular parameters of the patient and result in a change in the breathing pattern, e.g. due to an alter oxygen or carbon dioxide respiration in the respiratory system of the patient.

Accordingly, the system directly measures both the activity status of the patient and the actual respiratory parameters, e.g. a change of a breathing pattern by means of the activity sensor unit and the sensor for measuring the respiratory parameter. For example, the latter sensor may be arranged in a line or tube used to deliver a medical gas to a patient and may particularly be arranged at an end of said line in proximity of or even arranged within a coupled patient interface. Accordingly, an exact measurement of e.g. the breathing pattern may be obtained. Said sensor may hence be configured as a pressure sensor or flow sensor to provide the required measurement.

The activity level of the patient is hence factored into the measurement of the respiratory parameter, i.e. the measured respiratory parameter is evaluated as a function of the activity level, such that the exacerbation may be predicted more accurately.

Preferably, the activity sensor unit is adapted for distinguishing at least two activity levels, which may correspond to the patient being at rest and the patient performing at least one activity. It is further possible for the activity sensor unit to be able to distinguish different activities, wherein each activity may be assigned to a respective activity level. Measuring the respiratory parameter as a function of the activity level preferably includes measuring the respiratory parameter when the patient's activity level corresponds to either of the activity levels and/or during a change from one to another activity level of the patient. The activity level may furthermore also correspond to an intensity of an activity and/or a change in intensity of activity.

The indication of the exacerbation prediction is performed in the evaluation unit, which may be provided as a separate device or as an integrated part of another system component, e.g. a control unit.

Determining the current health level and/or the long-term health level on the basis of the measured vital parameters as a function of the corresponding measured activity level may include applying an algorithm to the measured vital parameters

In order to determine a potential development of an exacerbation, the evaluation unit may preferably be adapted for mathematically processing the measured respiratory parameter values as a function of the measured activity level, e.g. by means of a corresponding algorithm. For example, the evaluation unit may comprise a processing unit and/or a storing unit in order to determine the development of a patient's health status and compare said status with baseline values for the respective parameters, which are factored according to the measured activity level. In case of a deviation from such calculated tolerance levels, the evaluation may then indicate an alarm corresponding to a potential development of an exacerbation.

Furthermore, to provide an improved measurement or indication of an exacerbation prediction, the measurement values may be compared with a data of the patient corresponding to a normal health state and obtained in the absence of an acute exacerbation and/or in the absence of an upcoming acute exacerbation or recovery from a recent one. Accordingly, the measurements may be compared with normalized data for the respective patient, wherein only a predefined deviation of said normalized data may indicate the potential development of an exacerbation.

Preferably, the evaluation unit is configured to determine a physiological parameter of the patient based on the measured activity level and the measured respiratory parameter of the patient, wherein the evaluation unit is configured to indicate the probability of an exacerbation based on the determined physiological parameter of the patient.

For example, the physiological parameter may comprise e.g. a determined oxygen respiration, blood oxygenation level, and/or a respiratory function based on a determined respiratory parameter. Depending on the configuration of the evaluation unit and the measured respiratory parameter, the evaluation unit may also be configured to detect the onset of a disorder, e.g. an impaired lung function due to, for example, a developing infection or inflammation, which directly affects the respiratory parameter.

To determine the physiological parameter the evaluation unit is furthermore preferably configured to evaluate the measured respiratory parameter comprising a breathing pattern or rhythm, an exhalation and/or inhalation time, a breathing volume, a dynamic flow measurement, a breathing or maximal lung capacity, and/or a breathing pulse, preferably over a predefined time and/or at predefined intervals.

Accordingly, various characteristics may be determined and e.g. a change of said characteristics detected by the evaluation unit may provide a trend or course of the patient's physiological parameter. Furthermore, the evaluation of the parameter may include an averaging, such that measuring values that occur infrequently and may be considered as outliers do not immediately alter the determined patient's physiological parameter.

The system hence provides a more reliable and higher quality data collection, thereby allowing better capturing of e.g. adherence data and hence providing a greater accuracy in determining potential exacerbation conditions. Furthermore, the system may be implemented in existing adherence devices, such that a cost effective method for predicting exacerbations may be provided.

To differentiate between various activity levels, the evaluation unit is furthermore preferably configured to determine a phase of the activity, preferably a start of an activity and/or an end of an activity, to determine a slope of the respiratory rate based on the measured respiratory parameter, and to indicate a probability of an exacerbation based on the slope of the respiratory rate at the determined phase of the activity.

For example, the normal physiological response of a patient when they begin an activity, i.e. from a seated position to the start of walking, is that the respiratory rate will increase to meet the increased oxygen demand required by the human body. By the same token, the respiratory rate will decrease once the activity is stopped.

When the patient's health condition is stable, it is hence expected that the rate of increase in respiratory rate (dRR/dT)a, i.e. at the start of activity, and the rate of decrease in respiratory rate (dRR/dT)r, i.e. when activity is stopped, will be of similar behavior when compared to similar activities. However, when the patient's health condition starts to deteriorate, for example with the onset of a chest infection or other worsening of the patient's condition, it is considered that the magnitude of (dRR/dT)a will increase, i.e. the patient will breath more quickly, while the respiratory rate following cessation of activity will take longer to reach the pre-activity respiratory rate.

Accordingly, by evaluating the slope of the respiratory rate based on the measured respiratory parameter and by furthermore differentiating between a phase of the activity, e.g. between a first activity level corresponding to the patient being in rest and a second activity level corresponding to the patient performing an activity, the evaluation unit may provide a more differentiated and accurate indication of an exacerbation prediction.

The evaluation unit may furthermore be configured to determine a shape of a respiratory profile, preferably a change of the shape of the respiratory profile, to determine the physiological parameter.

For example, the respiratory profile may be analyzed to determine a variation curve of the shape of the respiratory profile as a function of the measurement time. The shape of the respiratory profile and/or the respective variation curve may e.g. be determined from a maximum value of a respiratory parameter measured during the initial activity level, the respiratory parameter measured at a first time during the final activity level, and a the respiratory parameter measured at a second time during the final activity level. In particular, the first time (second time) may be the time it takes for the respiratory parameter to drop from the maximum value to a predefined percentage of the maximum value. Furthermore, the shape may be analyzed by providing a representation of a volumetric or flow measurement and change profile during an inhalation and/or exhalation.

The change of the shape of the respiratory profile during a change in the patient's activity level may give deeper insight in the patient's current health level and in particular may be more sensitive to a deterioration of the patient's health level. For example, if the change in activity level corresponds to the end of an exercise, a steeper slope may correspond to a better current health level than a flatter slope.

In particular, by analyzing the shape of the respiratory profile, the prediction of an exacerbation may be further improved. For example, while the shape of the breathing or respiratory profile in resting conditions may not significantly deviate from a corresponding shape for a patient experiencing a beginning worsening in their condition, the shape of the breathing profile may change more markedly with activity, e.g. when walking stairs or even during a normal walking routine. To provide a further evaluation of the activity level, the system is preferably adapted to determine at least one of the following parameters relating to the activity level: a time of the day; whether the measured activity level (AL) corresponds to walking, running, or cycling to accordingly measure a walking or cycling speed; and whether the measured activity level (AL) corresponds to an activity that includes a change in altitude or walking up and/or down steps to accordingly measure a walking speed and altitude gain.

It may thus be possible to determine whether it is day or night, which may give additional indications for the current activity level and/or the current health level of the patient. In this case, the system preferably comprises a GPS sensor and/or a clock. In addition or as an alternative, if the measured activity level corresponds to an activity that includes walking, the system may be adapted to determine the walking speed. The walking speed may be determined e.g. by use of a GPS sensor and/or with an internal clock of the system in combination with a motion sensor. Further, in addition or as an alternative, if the measured activity level corresponds to an activity that includes a change in altitude, the system may be adapted for determining the walking speed and the altitude gain. For determining the altitude gain, the activity sensor unit may e.g. comprise an altimeter and/or a GPS sensor.

Preferably, the activity sensor unit comprises at least one of the following sensors: motion sensor; accelerometer; gyroscope, GPS sensor, in particular GPS activity sensor; step counter; altimeter; passive transponder, in particular RFID chip; internal clock; active transponder; position sensor; body temperature sensor.

Measuring activities with at least one of these sensors may hence allow for an accurate determination of the patient's activity level and/or of the patient's position, for example, whether the patient is standing, sitting or lying. For example, the activity sensor unit may comprise a step counter that is adapted for counting the number of the climbed steps and the climbing speed - alone or in combination with further sensors, as for example a motion sensor and/or a GPS sensor - to determine, how fast a patient is climbing stairs. If the activity sensor unit comprises a GPS sensor, said sensor is preferably used for calibrating other sensors of the system. As an example, the activity sensor unit may comprise an internal clock and/or an altimeter, wherein the absolute time and/or the absolute height may be calibrated by use of the GPS sensor. This allows for measuring activities even if no GPS signal is available as well as reducing the noise of the signal of the activity sensor unit since the signal of the GPS sensor may fluctuate.

To provide an even further differentiation of the measured values and an even higher accuracy of the exacerbation prediction, the evaluation unit may include further parameters relating to the patient's treatment. Accordingly, the system preferably further comprises a sensor arranged to be in fluid communication with a medical gas flow between a medical gas source and the patient and configured to measure a flow characteristic of a medical gas delivered to a patient, preferably a flow rate and/or a flow volume and/or a change thereof, in particular over a predefined time and/or at predefined intervals, wherein the evaluation unit is configured to indicate the probability of an exacerbation based on the measured flow characteristic.

For example, the system may comprise a flow sensor or pressure sensor, which may be arranged in a line or tube used to deliver a medical gas to a patient and may particularly be arranged at an end of said line, either in proximity of or even arranged within a coupled patient interface, or in proximity of a medical gas source to be coupled. Accordingly, an exact measurement of e.g. the flow rate and fluctuations thereof may be provided, such that the evaluation unit may evaluate the measured respiratory parameter and the activity level in view of a current treatment or therapeutic measure.

To provide a facilitation of the measurement and analysis provided by the evaluation unit, the evaluation unit may furthermore be configured to determine a differential pressure in a medical gas flow between a medical gas source and the patient, wherein the evaluation unit is configured to determine the flow direction based on the determined differential pressure and is configured to accordingly switch the at least one sensor based on the determined flow direction.

As various components of the system may be arranged at different positions, depending on e.g. the requirements of the system and the dimensions of said components, said components, e.g. monitoring units and connecting tubes, may be coupled in various orientations. Accordingly, in order to retrieve the measurement values from a correct sensor, the evaluation unit may determine a flow direction in e.g. a tubing or line, in order to associate the correct sensor with the parameter to be measured. Hence, by determining the differential pressure, an orientation and hence a position of a respective sensor may be determined in a fast and simple manner.

The determining of the differential pressure may be provided by having e.g. a first sensor and a second sensor in a respective component, which are in fluid communication and may be arranged at opposite ends of e.g. a fluid channel and may be configured to measure a pressure. The evaluation unit may then determine a virtual differential pressure measurement by creating an artificial impedance in the channel between the first sensor and the second sensor, wherein the exact positions of the sensors and the characteristics of the channel are preferably known. In a connected or coupled state, i.e. in a system with a medical gas source and a patient interface, the pressure measured at the sensor located nearest to the medical gas source, e.g. at a gas inlet, will be higher than the measured pressure at the other sensor, e.g. at a gas outlet or a gas inlet of a patient interface, such that a flow direction may be determined based on the calculated differential pressure and/or the respective pressure at each of the sensors.

Alternatively, or in addition, a differential pressure sensor may be provided in a respective component, which measures a pressure difference at two sides of the sensor. The differential pressure sensor may be arranged at a respective end of a fluid channel, but may also be arranged at a central position thereof. Accordingly, the differential pressure provides either a negative or a positive measurement value depending on the flow direction with respect to the differential pressure sensor. Accordingly, the evaluation unit may directly determine the flow direction based on said measurement value.

To provide even further information that may be factored in to the exacerbation prediction, the evaluation unit may include further parameters relating to the patient's treatment. Accordingly, the system preferably further comprises a detector unit configured for measuring at least one vital parameters of the patient, preferably as a function of the measured activity level, wherein the evaluation unit is configured to indicate the probability of an exacerbation based on the measured vital parameter.

The vital parameters that may be detected by the detector are preferably selected from the group of: heart rate; heart rate variability; ECG derived QRS durations; breath rate; oxygen and/or carbon dioxide saturation; skin and/or body temperature; ECG; blood pressure; plethysmography traces; derived oxygen consumption and/or effort surrogates; sleep duration; sleep depth; duration, frequency, quality and/or temporal grouping of cough.

The afore-mentioned values are particularly advantageous for monitoring a COPD patient. These values as well as calculated values derived from them can be combined with data from the patient's health history and/or the patient's general health status. As an example, the heart rate and/or the heart rate variability may be measured during a change of the activity level to determine the current health level of the patient. If a patient's activity level is changed from a high activity, such as running, to a low activity, such as sitting, the heart rate drops. This drop in the heart rate may be slower in the case of a deterioration of the patient's health status, thereby indicating a higher risk for an acute exacerbation.

The provision of such vital parameters may hence provide further information related to the activity level and the health status of the patient. Such information may generally be obtained by non-invasive methods, e.g. by use of optical sensors or pulse oximeters, thereby providing no significant burden to the patient while at the same time providing a further differentiation of the measured values, thereby further increasing the accuracy of the exacerbation prediction.

To provide an increased flexibility of the system and an improved movability of the patient, the system preferably comprises a wearable device that comprises at least the activity sensor unit, which is integrated in the wearable device. The wearable device furthermore comprises a communication device for communicating the measured activity level of the patient to the evaluation unit. Furthermore, it may be provided that the evaluation unit is integrated in the wearable device.

In addition, it may be provided that the wearable device comprises the detector unit, wherein the communication device is configured for communicating the measured vital parameters to the evaluation unit.

The wearable device may comprise a wristband, a wearable cuff, electrodes, a microphone, a wearable ECG and/or further sensors for measuring the vital parameters. Preferably the wearable device is configured as a smart wristband or smartwatch. The evaluation unit may be integrated into the wearable device or may be a separate device, such as an, in particular external, computer, a smartphone, a smartwatch, and/or a tablet computer. Although the components are preferably fully integrated in the wearable, it is also to be understood that a component is "integrated into the wearable device", if said component is also a wearable device which is carried by the patient. In particular, a component being "integrated into the wearable device" may mean, that the component is part of the seam wearable device as the activity sensor unit or detector unit. For example, the evaluation unit may be the patient's smartphone, tablet computer and/or smartwatch that is connected to the detector unit via a Bluetooth and/or Wi-Fi connection.

The communication device preferably is a wireless communication device, such as a Bluetooth and/or a Wi-Fi antenna. Preferably, the communication is provided via Bluetooth Low Energy (BLE). The communication device may alternatively be a tethered communication device, for example an USB connector or a wire soldered to the activity sensor unit or detector unit and the evaluation unit. It is further possible that the communication device is the Wi-Fi and/or Bluetooth unit of the patient's smartphone, tablet computer and/or smartwatch. The evaluation unit may then be an external computer. In general, the external computer may be positioned at the healthcare service provider, which may check the results of the system and may contact the patient if an alert is triggered by the system.

Preferably, the wearable device is a portable device such that remote monitoring may be performed continuously and may not restrain a patient in his daily routine. The success of a patient management system highly depends on the acceptance and adherence of the patient. The more vital parameters that need to be measured by different detectors, the higher the probability of drop outs or lack of adherence of the patient, resulting in losses of data, predictability and, finally, in a lack of prevention of hospitalization. Therefore, the wearable device is preferably a small device with wireless connections.

Furthermore, further integrating the evaluation unit into the wearable device allows for a fast feedback of the results of the comparison conducted by the evaluation unit to the patient. The evaluation unit may further comprise a display device for displaying the results of the comparison.

This may accordingly increase the acceptance by the patient and hence may lead to an increased adherence, thereby reducing the potential development of exacerbations. In particular, only the wearable device has to be carried around by the patient and/or the patient does not have to be contactable by an external healthcare service provider. This may be particularly useful if the patient is not at home but, for example, on vacation, on a trip, out shopping or at work.

By the same token, the integration in a wearable and/or a corresponding transmission or communication of the data may provide that the patient's health may be monitored, e.g. during a patient recovery following surgery, while the patient is situated in other locations such as a hospital. Furthermore, such implementation may be used for monitoring the progression of disease, for example during the 6-minute test, which is used to diagnose COPD.

The wearable device is preferably adapted to record the target vital parameters and activity levels in a 24/7 manner (i.e., around the clock) according to pre-defined intervals for measurement close enough to not miss relevant patient information. Combining continuous monitoring with a wearable device is particularly advantageous since the background noise level may be significantly reduced by a high number of measurements. Specifically, in an everyday uncontrolled situation, the type and intensity of an activity is unknown. Therefore, it is very difficult to discriminate between values recorded while the patient is performing a high activity, such as climbing stairs, or values recorded while the patient is performing a low activity, such as calmly walking in between two rooms of his home.

The wearable device can also provide derived parameters on the basis of raw data measured by the sensors and analyzed, processed, combined and/or reduced on the basis of predetermined rules or principles. For example, acceleration raw data measured by an acceleration sensor can be used to derive patient activity monitoring parameters as well as breathing rate monitoring parameters. Acoustic data measured by a microphone can also be used to derive breathing rate monitoring parameters and additionally acoustic profiles that are qualitatively or quantitatively representative of the quality of breathing (e.g. inspiration to expiration ratios, wheeze, cough, crackles and/or other added acoustic phenomena derived from the thoracic, cervical, buccal and oronasal tracts).

By the same token, the sensor for measuring a respiratory parameter may be arranged e.g. in a nasal mask or prong or facial mask and be in communication with the evaluation unit via a respective transmitter. This has the advantage that e.g. a breathing pattern and fluctuations thereof, with or without having a medical gas applied, may be measured while the patient is less restricted in its movement and movability.

To further increase the safety of the system and in order to provide e.g. a corresponding measure, when an exacerbation probability is increased, the system may furthermore comprise a monitoring unit, wherein the monitoring unit is in communication with the evaluation unit and is configured to compare at least the measured respiratory parameter and the measured activity level with a corresponding threshold or control logic stored in the monitoring unit to indicate a probability of an exacerbation. Furthermore, the monitoring unit is configured to automatically output a signal, when said threshold is exceeded and/or based on a value of said control logic.

Preferably, the monitoring unit comprises a channel configured to be in fluid communication with a medical gas source at a first end and with a patient interface at a second end and having at least the sensor for measuring the respiratory parameter arranged at one end of the channel. For example, the monitoring unit may be configured as a connecting line to be coupled between a medical gas source and a patient interface. When the monitoring unit is provided with an indication of the evaluation unit that an exacerbation probability is present, e.g. a predefined deviation from a baseline is exceeded, the monitoring unit may e.g. output an alert or alarm, e.g. a warning light and/or an acoustic warning signal.

To increase the data availability and to allow that said data or measurements may be accessed at another time point, the system preferably comprises a storage medium, preferably a database, to store the measured respiratory parameter, the measured activity level, and/or the indicated probability of exacerbation. Said storage medium is preferably configured as an external storage medium, wherein the system comprises a communication device configured to communicate the measured respiratory parameter, the measured activity level, and/or the indicated probability of exacerbation to the external storage . Alternatively, or in addition, the storage medium may provided locally, e.g. in the evaluation unit.

For example, the evaluation unit may comprise a storage unit, such as a memory chip. Storing the measured respiratory and vital parameters as well as the corresponding activity levels of the patient may be particularly useful for determining the long-term health level of the patient. For example, the determined current health level of the patient may be stored in the evaluation unit and averaged with subsequently measured current health levels.

The respective data may be communicated by means of e.g. via Bluetooth, Bluetooth Low Energy (BLE), ZigBee, RFID, near-field communication (NFC), WAN, WLAN, infrared signaling, and/or any other broadcasting means. Furthermore, the database may be provided either locally in a computer system or in cloud storage.

The communication of the respective data to a remote location at least has the advantage that the activity and respiratory data can either be measured and analyzed or evaluated on the same device in the system or can be communicated from multiple sources and analyzed remotely, for example, in an analysis database. When either the device or the database finds that the respiratory rate does not follow an expected or predefined behavior, an alert to either the patient or clinical staff may be sent, indicating that the patient is approaching an exacerbation event.

According to a further aspect of the invention, a method for predicting an exacerbation of a health condition of a patient is suggested, comprising the steps of:
- providing a system according to any of the preceding claims to a patient;
- measuring the patient's activity levels using an activity sensor unit;
- measuring a respiratory parameter of the patient using a sensor arranged to be in fluid communication with a medical gas flow between a medical gas source and the patient; and
- indicating a probability of an exacerbation based on the measured activity level and the measured respiratory parameter using an evaluation unit in communication with the activity sensor and the sensor.

Preferably, the method also comprises the step of determining a physiological parameter of the patient based on the measured activity level and the measured respiratory parameter of the patient using the evaluation unit, wherein the step of indicating the probability of an exacerbation is based on the determined physiological parameter of the patient, and/or the step of measuring at least one vital parameter of the patient using a detector unit, wherein the step of indicating the probability of an exacerbation is based on the measured vital parameter.

The method is preferably conducted with the system according to the present invention and/or the system preferably is adapted for performing the method according to the present invention. That is to say, all features that are disclosed with reference to the system are also disclosed for the method and vice versa. Accordingly, various features and other functions of the described system may be provided in accordance with the suggested method, which are not described in further detail merely for redundancy reasons.

### Brief description of the drawings

The present disclosure will be more readily appreciated by reference to the following detailed description when being considered in connection with the accompanying drawings in which:
Figure 1 is a schematic view of a system having an activity sensor unit and a sensor for measuring a respiratory parameter in a medical gas treatment application; and
Figure 2 is a schematic view of a system with integrated components in a wearable device and a database.

### Detailed description of preferred embodiments

In the following, the invention will be explained in more detail with reference to the accompanying figures. In the Figures, like elements are denoted by identical reference numerals and repeated description thereof may be omitted in order to avoid redundancies.

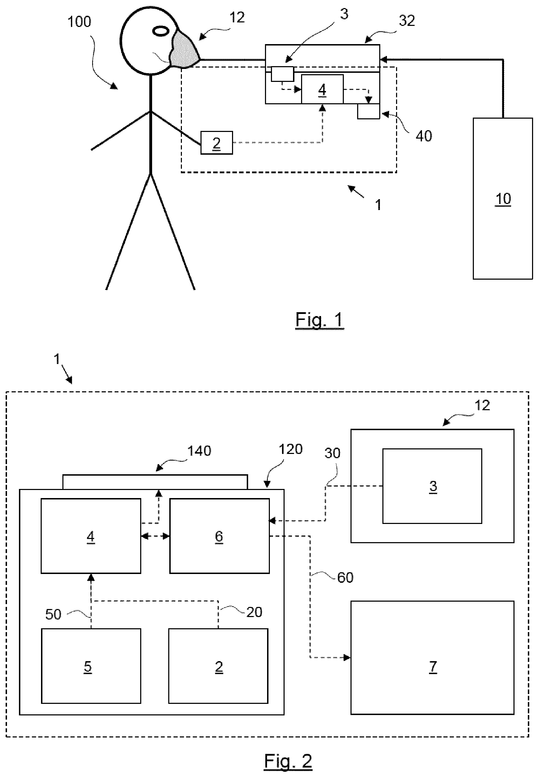

In Figure 1 a system 1 for predicting an exacerbation of a health condition of a patient 100 is schematically shown. The system 1 is generally depicted in a medical gas application setting to accordingly provide a treatment to the patient 100, i.e. by means of a medical gas delivery. Said medical gas is provided by a medical gas source 10, which may e.g. comprise oxygen or a mixture of medical grade air enriched with e.g. oxygen, helium, and/or nitric oxide. The medical gas source 10 comprises a pressure regulator and a valve or a valve integrated pressure regulator (not shown) to deliver the medical gas 10 via a corresponding outlet, indicated by the arrow, in direction to the patient 100. A patient 100 receiving long-term oxygen therapy will generally be prescribed a flow rate in the region of 1 - 15 liters per minute. This can take the form of continuous flow, or may be delivered via a demand valve or an oxygen concentrator which itself delivers an equivalent flow in a series of pulses.

At the patient 100 a patient interface 12 is provided, which is depicted here as a facial mask or nasal prong. However, it is to be understood that the patient interface 12 may have any other configuration, as required for the respective treatment of the patient 100. Between the patient interface 12 and the medical gas source 10 a coupling 32 is provided, which hence provides a fluidic coupling between said components. The coupling 32 hence defines an inner fluid channel through which the medical gas flows in the direction from the medical gas source 10 to the patient interface 12. The coupling 32 may be configured to be essentially comprised of a flexible medical tube, but may also comprise other configurations, e.g. as a monitoring device.

Within the coupling 32 and arranged to be in fluid communication with a medical gas delivered to the patient 100 is a sensor 3 for measuring a respiratory parameter. The sensor 3 is depicted to be arranged at a downstream end of the coupling 32, which is preferable in order to measure respiratory influences and fluctuations from the patient 100, e.g. due to a patient's respiration. However, said sensor 3 may also be provided or arranged at another position along the channel of the coupling 32, e.g. to measure the respiratory parameter more indirectly but sufficiently, e.g. at a more central position of the coupling 32 in order to provide for more robustness of the coupling 32 and the sensor 3.

The sensor 3 may hence provide a variety of respiratory parameters depending on the actual configuration. For example, the measured respiratory parameter may comprise a breathing pattern or rhythm, an exhalation and/or inhalation time, a breathing volume, a dynamic flow measurement, a breathing or maximal lung capacity, and/or a breathing pulse. At least one of said parameters may then be used to further determine a physiological parameter and is preferably measured over a predefined time and at predefined intervals.

The sensor 3 is in communication with an evaluation unit 4, which may hence receive the measured respiratory parameter. Said evaluation unit 4 is depicted within the coupling 32, but may also be provided outside of the coupling in another component or device in proximity of the patient 100 or within the system 1. The evaluation unit 4 according to the embodiment depicted in Figure 1 is configured as a microprocessor having a control logic stored therein.

In addition, the evaluation unit 4 is in communication with an activity sensor unit 2 in order to measure an activity level of the patient 100. Said activity sensor is carried by or applied to the patient 100 and may have various configurations, as described in the above. The activity sensor unit 2 according to the embodiment of Figure 1 is configured as a motion sensor, such that the evaluation unit 4 may accordingly receive the activity level in the form of a detected motion.

For example, the measured motion may indicate whether the patient 100 is at rest, e.g. when minimal movements are detected, or when a patient 100 is walking, e.g. when period movements are detected. By the same token, the detected motions may be evaluated to assess a position of the patient 100, i.e. whether the patient 100 is in a seated, upright standing, or lying position. Accordingly, the evaluation unit 4 may be provided with various informations relating to the activity level of the patient 100, wherein an active status or an inactive, i.e. rest status, may be considered to be the simplest forms.

Based on the received measured activity level and the measured respiratory parameter, the evaluation unit 4 may then evaluate the patient's health and accordingly indicate a probability of an exacerbation development. For example, an increased breathing in a resting state, as indicated e.g. by a measured increased breathing frequency by the sensor 3 and a measured inactivity, e.g. detection of minimal movements, of the activity sensor unit 2, may indicate that a patient 100 is experiencing breathing difficulties. When said breathing frequency is further increased over time without detecting a change in the activity level of the patient, the evaluation unit 4 may determine that an exacerbation may develop, e.g. when a predefined threshold from a baseline or tolerance level is exceeded. Said threshold or baseline is preferably patient-specific, but may also be provided as population data for a respective patient class, e.g. a group of patient's having a similar disorder, age, weight, and/or co-morbidity.

Accordingly, should the evaluation unit 4 indicate a predicted exacerbation, the evaluation unit 4 may accordingly provide a signal via the signaling device 40. The signaling device 40 may e.g. be configured to output a light, e.g. via a corresponding LED or set of LEDs, but may also be configured to output an acoustic warning signal, e.g. an alarm. Hence, a patient 100 and/or medical personnel may be immediately alerted, when a patient 100 is experiencing a worsening of a disorder and/or is at risk of collapsing, e.g. due to persisting circulation and/or respiration problems. By means of the evaluation unit 4 and the signaling device 40, an intervention may hence immediately be provided, thereby remedying the worsening of the patient's condition and potentially avoiding a hospital admission.

In Figure 2 a schematic view of a system 1 with integrated components in a wearable device 120 is shown in communication with a database 7. Accordingly, several components corresponding to those of Figure 1 are now comprised in the wearable device 120, which is schematically depicted as a smartwatch, and the communication between said components is described in further detail in view of the Figure 2 embodiment.

Accordingly, the wearable device 120 comprises the activity sensor unit 2, which is in direct communication with the evaluation unit 4, as indicated by the arrow, and which is also arranged within the wearable device 120. The activity sensor unit 2 is configured to measure an activity level 20 of the patient. The activity sensor unit 2 according to Figure 2 is configured as an accelerometer, such that the evaluation unit 4 may be accordingly provided with a motion, an absolute acceleration, and a determined speed, e.g. a walking speed of the patient. Accordingly, a more detailed activity level 20 is communicated to and/or determined by the evaluation unit 4.

Also provided in the wearable device 120 is a detector unit 5, which is configured to measure a vital parameter 50 of the patient. The detector unit 5 may comprise any configuration in order to measure a required vital parameter and is configured as an electrode arrangement at an inner surface of the smart watch to measure a heart rate or pulse, e.g. at regular intervals or continuously, as a vital parameter 50. Said vital parameter 50 is then communicated to the evaluation unit 4, which then determines an indication of a probability of an exacerbation of the patient based on the provided activity level 20 and the additionally provided vital parameter 50.

In addition, the evaluation unit 4 is provided with the respiratory parameter 30 as measured by the sensor 3. Here, the sensor 3 is arranged in the patient interface 12 and is configured as a pressure sensor to measure pressure fluctuations due to the patients respiration. The respiratory parameter 30 is provided via a wireless interface to the evaluation unit 4 provided by a communication device 6, which is configured as a Bluetooth Low Energy communication device 6. However, it is to be understood that any communication device may generally be implemented, which provides a sufficient transmission range, e.g. RFID, NFC, or Wi-Fi.

The evaluation unit 4 is hence provided with the respiratory parameter 30 via a corresponding communication link between the communication device 6 and the evaluation unit 4. In addition to the vital parameter 50 and the activity level 20, also the respiratory parameter 30 is factored into the prediction of the exacerbation, such that the embodiment according to Figure 2 allows that the exacerbation prediction may generally be based on more detailed information and more variables compared to the embodiment according to Figure 1. However, any of the general features of Figure 2 may likewise be implemented in the embodiment according to Figure 1, and vice versa.

Furthermore, although not specifically shown in Figure 2, the system 1 may comprise additional sensors that may provide additional information relating to the medical gas delivered to the patient. For example, a coupling device, as e.g. depicted in Figure 1, may be provided having an additional pressure sensor at an upstream end of a channel, such that a flow rate of the medical gas delivered to the patient may be measured and provided to the evaluation unit 4 via a corresponding communication. Accordingly, the patient's health status may be directly linked to a currently applied medical gas treatment, such that e.g. potential changes or fluctuations may be directly attributed to said treatment and a potential adjustment of said treatment may e.g. be performed.

Furthermore, the system 1 comprises a display 140, which is arranged at an outer surface of the wearable device 120 and is in communication with the evaluation unit 4. Accordingly, both the received data, e.g. the activity level 20, the vital parameter 50, and the respiratory parameter 30 may be displayed at the display to provide an instant feedback to the patient. Furthermore, the evaluation unit 4 may output a signal to be displayed on the display, e.g., when an exacerbation probability is increased, such that a patient and/or medical personnel may be accordingly alerted and e.g. a current activity is to be stopped or at least reduced. By the same token, the absence of such indication may assure the patient that even when e.g. experiencing small breathing difficulties, the patient is not at risk, i.e. the activity does not pose a safety hazard to the patient. Accordingly, the stress level of the patient may be reduced and the safety of the patient may be monitored effectively in real-time.

In addition, the wearable device 120 is in communication with a database 7 or other storage medium, such that the wearable device 120 may communicate data 60 to said database 7 via the communication device 6. The database may hence be provided at a remote location, such that the data 60 may e.g. be provided to medical staff monitoring the patient while the patient is situated at home or in a remote ward. The database may generally be part of a computer system, such that the provided data 60 may be further evaluated and analyzed. For example, the actual data may be compared with previous log entries of the patient or a log history, such that a more detailed assessment of a potential exacerbation may be provided. Accordingly, the database 7 may also provide that a remote monitoring is provided, thereby further increasing the safety of the patient while minimizing the required components in the wearable device 120.

By the same token, the evaluation unit 4 of the wearable device 120 may be provided at the external location in an alternative configuration, such that the dimensioning and sizing of the wearable device 120 and also the energy consumption thereof may be minimized. In order to provide the patient with the required feedback, the communication device 6 may be configured to also receive data from a remote location such as the database 7. Accordingly, information relating to the patient's health may hence still be displayed on the display 140 via a corresponding link to e.g. the communication device 6.

Both the evaluation unit 4 and the database 7 may hence be configured to provide a monitoring of the patient's condition or health. In addition, such monitoring may be provided by the coupling, as described in view of Figure 1. Accordingly, such monitoring unit preferably comprises a channel configured to be in fluid communication with a medical gas source at a first end and with a patient interface at a second end and having at least the sensor 3 for measuring the respiratory parameter arranged at one end of the channel. In such configuration the monitoring unit may either comprise the evaluation unit 4 or may be in communication with the evaluation unit 4 provided e.g. in the wearable device 120.

It will be obvious for a person skilled in the art that these embodiments and items only depict examples of a plurality of possibilities. Hence, the embodiments shown here should not be understood to form a limitation of these features and configurations. Any possible combination and configuration of the described features can be chosen according to the scope of the invention.

### List of reference numerals

- 1: System
- 10: Medical gas source
- 12: Patient interface
- 100: Patient
- 120: Wearable device
- 140: Display
- 2: Activity sensor unit
- 20: Activity level
- 3: Sensor for measuring a respiratory parameter
- 30: Respiratory parameter
- 32: Coupling device
- 4: Evaluation unit
- 40: Signaling device
- 5: Detector unit
- 50: Vital parameter
- 6: Communication device
- 60: Data
- 7: Database

## Claims

1. System (1) for predicting an exacerbation of a health condition of a patient (100), comprising:
- an activity sensor unit (2) for measuring activity levels (20) of a patient (100),
- a sensor (3) arranged to be in fluid communication with a medical gas flow between a medical gas source (10) and the patient (100) and configured to measure a respiratory parameter (30) of the patient (100), and
- an evaluation unit (4) in communication with the activity sensor (2) and the sensor (3),
wherein the evaluation unit (4) is configured to indicate a probability of an exacerbation based on the measured activity level (20) and the measured respiratory parameter (30).

2. System (1) according to claim 1, wherein the evaluation unit (4) is configured to determine a physiological parameter of the patient (100) based on the measured activity level (20) and the measured respiratory parameter (30) of the patient (100), wherein the evaluation unit (4) is configured to indicate the probability of an exacerbation based on the determined physiological parameter of the patient (100).

3. System (1) according to claim 2, wherein the evaluation unit (4) is configured to evaluate the measured respiratory parameter (30) comprising a breathing pattern or rhythm, an exhalation and/or inhalation time, a breathing volume, a dynamic flow measurement, a breathing or maximal lung capacity, and/or a breathing pulse, preferably over a predefined time and/or at predefined intervals, to determine the physiological parameter.

4. System (1) according to claim 3, wherein the evaluation unit (4) is configured to:
- determine a phase of the activity, preferably a start of an activity and/or an end of an activity, to determine a slope of the respiratory rate based on the measured respiratory parameter (30), and to indicate a probability of an exacerbation based on the slope of the respiratory rate at the determined phase of the activity; and/or
- determine a shape of a respiratory profile, preferably a change of the shape of the respiratory profile, to determine the physiological parameter.

5. System (1) according to any of the preceding claims, wherein the system (1) is adapted for determining at least one of the following parameters relating to the activity level:
- time of the day;
- whether the measured activity level (AL) corresponds to walking, running, or cycling to accordingly measure a walking or cycling speed;
- whether the measured activity level (AL) corresponds to an activity that includes a change in altitude or walking up and/or down steps to accordingly measure a walking speed and altitude gain.

6. System (1) according to any of the preceding claims, wherein the activity sensor unit (2) comprises at least one of the following sensors:
motion sensor; accelerometer; gyroscope, GPS sensor, in particular GPS activity sensor; step counter; altimeter; passive transponder, in particular RFID chip; internal clock; active transponder; position sensor; body temperature sensor.

7. System (1) according to any of the preceding claims, further comprising a sensor arranged to be in fluid communication with a medical gas flow between a medical gas source and the patient and configured to measure a flow characteristic of a medical gas delivered to a patient, preferably a flow rate and/or a flow volume and/or a change thereof, in particular over a predefined time and/or at predefined intervals, wherein the evaluation unit (4) is configured to indicate the probability of an exacerbation based on the measured flow characteristic.

8. System (1) according to any of the preceding claims, wherein the evaluation unit (4) is configured to determine a differential pressure in a medical gas flow between a medical gas source (10) and the patient (100), wherein the evaluation unit (4) is configured to determine the flow direction based on the determined differential pressure and is configured to accordingly switch the at least one sensor based on the determined flow direction.

9. System (1) according to any of the preceding claims, further comprising a detector unit (5) configured for measuring at least one vital parameter (50) of the patient, preferably as a function of the measured activity level (20), wherein the evaluation unit (4) is configured to indicate the probability of an exacerbation based on the measured vital parameter (50), wherein the detector unit (5) is preferably adapted for measuring at least one of the vital parameters (50) selected from the group of:
heart rate; heart rate variability; ECG derived QRS durations; breath rate; oxygen and/or carbon dioxide saturation; skin and/or body temperature; ECG; blood pressure; plethysmography traces; derived oxygen consumption and/or effort surrogates; sleep duration; sleep depth; duration, frequency, quality and/or temporal grouping of cough.

10. System (1) according to any of the preceding claims, wherein the system (1) comprises a wearable device (120), wherein the activity sensor unit (2) is integrated into the wearable device (120), and wherein the system (1) further comprises a communication device configured to communicate the measured activity level (20) to the evaluation unit (4), and/or wherein the evaluation unit (4) is integrated into the wearable device (120).

11. System (1) according to claim 10 appended to claim 9, wherein the detector unit (5) is integrated into the wearable device (120) and wherein the communication device (6) is configured to communicate the measured vital parameters (50) to the evaluation unit (4).

12. System (1) according to any of the preceding claims, further comprising a monitoring unit, preferably comprising a channel configured to be in fluid communication with a medical gas source (10) at a first end and with a patient interface at a second end and having at least the sensor (3) for measuring the respiratory parameter arranged at one end of the channel, wherein the monitoring unit is in communication with the evaluation unit (4) and is configured to compare at least the measured respiratory parameter(30) and the measured activity level (20) with a corresponding threshold or control logic stored in the monitoring unit to indicate a probability of an exacerbation and is configured to automatically output a signal, when said threshold is exceeded and/or based on a value of said control logic.

13. System (1) according to any of the preceding claims, further comprising a storage medium, preferably a database (7), to store the measured respiratory parameter (30), the measured activity level (20), and/or the indicated probability of exacerbation, wherein said storage medium preferably is configured as an external storage medium and wherein the system comprises a communication device (6) configured to communicate the measured respiratory parameter (30), the measured activity level (20), and/or the indicated probability of exacerbation to the external storage.

14. Method for predicting an exacerbation of a health condition of a patient, comprising the steps of:
- providing a system (1) according to any of the preceding claims to a patient (100);
- measuring the patient's activity levels (20) using an activity sensor unit (2);
- measuring a respiratory parameter (30) of the patient (100) using a sensor (3) arranged to be in fluid communication with a medical gas flow between a medical gas source (10) and the patient (100); and
- indicating a probability of an exacerbation based on the measured activity level (20) and the measured respiratory parameter (30) using an evaluation unit (4) in communication with the activity sensor (2) and the sensor (3).

15. Method according to claim 14, further comprising the step of:
- determining a physiological parameter of the patient (100) based on the measured activity level (20) and the measured respiratory parameter (30) of the patient (100) using the evaluation unit (4), wherein the step of indicating the probability of an exacerbation is based on the determined physiological parameter of the patient (100), and/or
- measuring at least one vital parameter (50) of the patient (100) using a detector unit (5), wherein the step of indicating the probability of an exacerbation is based on the measured vital parameter (50).
